# EUROPÄISCHE PATENTSCHRIFT

(11) **EP 0 416 255 B1**
(45) Veröffentlichungstag und Bekanntmachung des Hinweises auf die Patenterteilung: **04.01.1995**
(21) Anmeldenummer: 90113674.7
(22) Anmeldetag: 17.07.1990
(51) Int. Cl.: A01M 17/00, A61L 2/20

(54) **Verfahren zum Entwesen von Gebäuden**
Process for eradicating pests in buildings
Procédé pour exterminer les vermines de bâtiments

(30) Priorität: 06.09.1989 DE 3929637
(43) Veröffentlichungstag der Anmeldung: 13.03.1991
(73) Patentinhaber: KOHLENSÄURE-WERKE RUD. BUSE GMBH & CO., D-53557 Bad Hönningen (DE)
(72) Erfinder: Corinth, Hans-Gerd, Dipl.-Ing., D-5462 Bad Hönningen (DE); Reichmuth, Christoph, Dr. Dipl.-Ing., D-1000 Berlin 37 (DE)
(74) Vertreter: Liesegang, Roland, Dr.-Ing.

(56) Entgegenhaltungen:
- EP-A- 0 113 321
- EP-A- 0 320 012
- DE-A- 2 203 027
- DE-A- 2 602 034
- DE-A- 3 445 990
- DE-C- 3 225 515
- GB-A- 490 008
- US-A- 1 861 736

## Beschreibung

Die Erfindung betrifft ein Verfahren zum Entwesen von Gebäuden für die Lagerung und/oder Verarbeitung von organischen Produkten, insbesondere von Mühlen mit den Merkmalen des Oberbegriffs des Anspruchs 1. Bei einem bekannten Verfahren dieser Art (GB-A-490 008), das zur Beseitigung von Insekten in Räumen oder Möbeln dient, wird gemäß Verfahrensschritt (b) der Luftinhalt des Gebäudes mittels erwärmter Luft auf eine "geeignete" Temperatur (nämlich nicht weniger als 25°C (77°F)) gebracht. Dann wird für einen Zeitraum von 10 bis 15 Minuten Kohlendioxidgas eingeführt, um die Atmung der Insekten anzuregen. Anschließend wird dann zur eigentlichen Entwesung Schwefeldioxid eingeleitet und für zwei bis drei Stunden oder mehr in dem Raum belassen.

Gebäude müssen in bestimmten Zeitabständen entwest werden. Bei der Entwesung von beispielsweise Mühlen geht man bisher so vor, daß zunächst die Türen und Fenster der Mühle abgedichtet werden, worauf in die Innenräume der Mühle Methylbromid (CH₃Br) eingeleitet wird. Nach erfolgter Entwesung werden die Abdichtungen wieder entfernt und die Mühlenräume gelüftet. Der gesamte Vorgang vom Beginn des Abdichtens bis zum Abschluß der Lüftung dauert je nach Größe der Mühle in etwa drei Tage; das Entwesen wird deshalb zur Vermeidung eines Produktionsausfalls meist an verlängerten Wochenenden, beispielsweise über die Osterfeiertage, vorgenommen.

Diese Entwesung mit Methylbromid ist sehr wirksam, vergleichsweise kostengünstig und kann, bei der erwähnten Zeitwahl, ohne Produktionsausfall durchgeführt werden. Ein beträchtlicher Nachteil besteht jedoch darin, daß Methylbromid ein Giftstoff ist, so daß es bei mangelhafter Anwendung und/oder Entfernung zu einer Personengefährdung kommen kann und darüber hinaus, nach der Entwesung wird das Methylbromid in die Umgebung entlassen, zu einer beträchtlichen Belastung der Umgebung. Aus Gründen des Personen- und des Umweltschutzes besteht somit das Bedürfnis eines Ersatzes des Methylbromids durch ein ungefährliches und ungiftiges Entwesungsmedium.

Zum Entwesen von Tabakspeichern ist es an sich bekannt, den vorher abgedichteten Speicher einer Inertgasatmosphäre mit einem O₂-Gehalt von 0 bis 5 %, einem CO₂-Gehalt von 1 bis 20 %, Rest N für einen Zeitraum von 10 bis 35 Tagen bei einer auf 27°C angehobenen Temperatur auszusetzen (EP-A-0 113 321).

Es ist bekannt, daß eine Entwesung mittels Heißluft durchgeführt werden kann. Dabei muß aber die Heißluft eine Temperatur von zumindest 90 bis 100° C haben. Bei der Entwesung von oder in Metallbehältern ist dieses Heißluftverfahren durchaus brauchbar, bei Gebäuden, wie etwa Mühlen, dagegen, deren Einrichtungen und Installationen zumindest teilweise hitzeempfindlich sind, kann dieses Verfahren verständlicherweise nicht angewendet werden. Weiterhin ist bekannt, daß mittels einer Kohlendioxidatmosphäre entwest werden kann. Freilich führt dieses Verfahren nur dann zu vertretbaren Entwesungszeiten, also Zeiten von wenigen Tagen, wenn mit vergleichsweise hohen Drücken gearbeitet wird. Auch dieses Verfahren kann somit zum Entwesen von Mühlen nicht eingesetzt werden, weil eine druckfeste Abdichtung einer Mühle nicht möglich ist und bei Verwendung von Kohlendioxid unter Normaldruck die Entwesungszeit viel zu lange, etwa zwei bis vier Wochen, dauern würde. Von wissenschaftlichen Untersuchungen her ist zwar bekannt geworden, daß die Entwesungswirkung von Kohlendioxid auch dadurch gesteigert werden kann, daß man das Kohlendioxid auf höhere Temperaturen bringt. In der Praxis jedoch ist von dieser Erkenntnis bei der Entwesung von Gebäuden und Räumen der eingangs erwähnten Art kein Gebrauch gemacht worden, zum einen, weil man befürchtete, ähnlich hohe Temperaturen wie beim Heißluftverfahren anwenden zu müssen, zum anderen, weil wirtschaftliche Überlegungen dagegen standen; es erscheint aus Kostengründen nicht tragbar zu sein, in einer Mühle, deren Rauminhalt bis zu 100000 m³ betragen kann, die verständlicherweise nicht exakt abdichtbar ist und deren Einrichtung, Installationen und innere Wandflächen, die ein bevorzugter Aufenthaltsort von Schädlingen sind, beträchtlich erwärmt werden müssen, über mehrere Tage eine warme bzw. heiße Kohlendioxidatmosophäre aufrechtzuerhalten.

Aus der DE 32 25 515 ist ein Verfahren zum Untergassetzen von Gebäuden bekannt, bei welchem Flüssiggas von einem Vorrat in ein in dem Gebäude befindlichen, mit Absperrorganen und Düsen versehenen Verteilersystem gedrückt und an vorher festgelegten Stellen versprüht wird. Bei diesem für die Schädlingsbekämpfung bestimmten Verfahren wird jedoch mit hochgiftigen gasförmigen Verbindungen gearbeitet, also gerade mit solchen Gasen, wie sie aus Gründen des Umweltschutzes vermieden werden sollen.

Die DE-OS 22 03 027 offenbart ein Verfahren zum Desinfizieren begehbarer Räume mit Hilfe eines gasförmigen Desinfektionsmittels. Bei diesem insbesondere für Krankenhäuser bestimmten Verfahren werden Desinfektionsmittel zum Abtöten von Bakterien und Pilzarten verwendet; weder das Verfahren noch die Desinfektionsmittel eignen sich zur Befreiung der hier interessierenden Gebäude vom Käfern und dergleichen, also zu einer wirtschaftlichen Entwesung. Schließlich ist aus der DE 34 45 990 ein Verfahren zur Entwesung von pflanzlichen und tierischen Produkten bekannt, bei dem die Produkte in einen Autoklaven eingebracht und in diesem mit einem Gas, beispielsweise Kohlendioxid, unter Druck behandelt oder abwechselnd einem Vakuum und Gasdruck ausgesetzt werden. Dieses bereits vorab angesprochene Verfahren, das einen druckfesten Behälter voraussetzt, eignet sich nicht für die Entwesung von Gebäuden.

Aufgabe der vorliegenden Erfindung ist deshalb ein Verfahren zum Entwesen von Gebäuden für die Lagerung und/oder Verarbeitung von organischen Produkten, insbesondere Mühlen, das bezüglich Wirksamkeit, Wirtschaftlichkeit und Dauer dem Methylbromid-Verfahren im wesentlichen entspricht, auch bezüglich der Schonung des Gebäudes und seiner Einrichtungen, im Gegensatz zum Methylbromid-Verfahren aber ungefährlich und umweltfreundlich ist. Die Lösung dieser Aufgabe ergibt sich aus den Merkmalen des Patentanspruchs 1.

Gemäß der Erfindung wird also zunächst das Gebäude, beispielsweise Mühle, abgedichtet, wobei es genügt,wenn bei einem der Außenatmosphäre entsprechenden inneren Luft- bzw. Gasdruck eine Abdichtung in etwa gewährleistet ist. Selbstverständlich wird das Verfahren umso wirtschaftlicher, je besser diese Abdichtung ist, jedoch hängen die Möglichkeiten in der Praxis von den jeweiligen Umständen ab. Nach dieser Abdichtung wird dann durch ein vergleichsweise kostengünstiges Medium die Gebäudewandung vorgewärmt, d.h. dafür Sorge getragen, daß zumindest die inneren Oberflächenbereiche der Wandungen und die Einrichtungen eine Temperatur von zumindest 27 bis 30° C aufweisen. Diese Vorerwärmung ist sehr wesentlich, weil sich erfahrungsgemäß Schädlinge gerade in Ritzen und Ecken bevorzugt aufhalten. Am einfachsten und kostengünstigsten ist es, in den einzelnen Mühlenräumen Lufterwärmer aufzustellen; es ist aber auch möglich, erhitzte Luft von außen her in die Mühle einzuleiten.

Wenn dann die Mühlenräume ausreichend "durchgewärmt" sind, wird die in der Mühle befindliche warme Luft weitgehend durch eine warme Kohlendioxidatmosphäre ersetzt. Durch die Vorerwärmung und durch das ebenfalls warme Kohlendioxid wird erreicht, daß die Schädlinge infolge temperaturaktivierter organischer Funktionen das Kohlendioxid sehr stark in sich aufnehmen; dabei ist auch die Löslichkeit des Kohlendioxids in den Körperflüssigkeiten der Schädlinge erhöht. Die Folge davon ist, daß die Entwesung in einer brauchbaren Zeit erfolgt, d.h. die Schädlinge in wenigen Stunden, spätestens in wenigen Tagen, tot sind. Die Erwärmung des Kohlendioxids und die Aufrechterhaltung der Temperatur während der Entwesungszeit können ebenfalls durch innerhalb der fraglichen Räume aufgestellte Erhitzungsgeräte bewirkt werden, jedoch ist es vorzuziehen, das Kohlendioxid in bereits erwärmtem Zustand in die Mühle einzuleiten. Die Einleitung sollte möglichst im untersten Mühlenbereich erfolgen; das Kohlendioxid, das ja schwerer als Luft ist, verdrängt dann langsam die Warmluft nach oben und, beispielsweise durch eine offene Dachluke, nach außen. Im allgemeinen muß während der Entwesungszeit warmes Kohlendioxid nachgeliefert werden, wobei Häufigkeit und Menge der Nacheinführung von der Gas- und Wärmedichtheit der Mühlenwandungen abhängt.

Nach Ablauf der Entwesungszeit, beispielsweise nach zwei Tagen werden die Abdichtungen entfernt, und die Mühle wird "gelüftet", wozu es im allgemeinen genügt, alle Türen und Fenster zu öffnen.

Die Kohlendioxid-Luft-Atmosphäre sollte möglichst reich an Kohlendioxid sein; ein Kohlendioxidanteil von zumindest 60% ist vorzuziehen. In diesem Fall kommt man dann bei einer Temperatur zwischen 30 und 40° C des Kohlendioxids mit einer Einwirkungszeit zwischen 3 Tagen und einem halben Tag aus, arbeitet man beispielsweise mit einer Kohlendioxidatmosphäre von 60 bis 80% CO₂ und einer Temperatur von 39° C, dann kann der Entwesungsvorgang, einschließlich der Abdichtung, der Luft-Vorerwärmung auf zumindest 39° C, der eigentlichen Entwesung mit Kohlendioxid, der Entfernung der Abdichtung und der Luft, innerhalb eines verlängerten Wochenendes stattfinden, ähnlich wie im Fall der Verwendung des giftigen Methylbromids. Auch Wirksamkeit und Wirtschaftlichkeit sind durchaus mit dem Methylbromid-Verfahren vergleichbar. Audererseits aber besteht nach dem "Lüften" keine Gefahr für die das Mühleninnere wieder betretenden Personen, und die Außenatmosphäre wird, weil Kohlendioxid ein neutrales Gas ist, nicht belastet.

Selbstverständlich kann das beschriebene Verfahren zahlreiche Abwandlungen erhalten, ohne den Bereich der Erfindung zu verlassen. Dies gilt insbesondere für die Dauer der einzelnen Verfahrensstufen, für die Temperaturen, soweit sie sich in den beanspruchten Grenzen halten, und für die Mittel zum Erreichen und Erhalten der gewünschten Temperaturen. Auch die Anwendung ist selbstverständlich nicht auf Mühlen beschränkt, vielmehr kann das erfindungsgemäße Entwesungsverfahren auch bei anderen Gebäuden und Räumen Anwendung finden, in denen sich Maschinen befinden, an den Wänden elektrische Kabel verlaufen und dergleichen.

## Patentansprüche

1. Verfahren zum Entwesen von Gebäuden für die Lagerung und/oder Verarbeitung von organischen Produkten, insbesondere von Mühlen, wobei
a) der Innenraum des Gebäudes gegenüber der Außenatmosphäre abgedichtet wird;
b) der Luftinhalt des Gebäudes auf eine erhöhte Temperatur gebracht wird;
c) Kohlendioxid in den Innenraum des Gebäudes eingebracht wird;
d) die Abdichtung des Gebäudes wieder entfernt und der Innenraum des Gebäudes entlüftet werden
dadurch **gekennzeichnet**, daß
e) der Luftinhalt des Gebäudes auf eine Temperatur zwischen 35 und 50°C gebracht und so lange auf dieser Temperatur gehalten wird, bis die Innenwände des Gebäudes eine Temperatur von zumindest 27°C angenommen haben;
f) der Luftinhalt des Gebäudes durch eine Kohlendioxidluftatmosphäre mit einem Anteil an Kohlendioxid von mindestens 60 % einer Temperatur zwischen 30 und 40°C ersetzt wird und
g) die Kohlendioxidluftatmosphäre bei dieser Temperatur während einer Zeitspanne von wenigen Stunden bis zu wenigen Tagen aufrechterhalten wird.

2. Verfahren nach Anspruch 1, dadurch **gekennzeichnet**, daß die einen Anteil von überall zumindest 60 % Kohlendioxid aufweisende Kohlendioxidluftatmosphäre auf eine Temperatur zwischen 32 und 38°C gebracht und bei dieser Temperatur eineinhalb bis zweieinhalb Tage aufrechterhalten wird.

## Claims

1. Process for eradicating pests from buildings for storing and/or processing organic products, in particular mills, wherein
a) the inner chamber of the building is sealed with respect to the external atmosphere;
b) the air content of the building is brought to a high temperature;
c) carbon dioxide is introduced into the inner chamber of the building;
d) the sealing of the building is removed again and the inner chamber of the building is ventilated,
characterised in that
e) the air content of the building is brought to a temperature of between 35 and 50°C and is kept at this temperature until the internal walls of the building have reached a temperature of at least 27°C;
f) the air content of the building is replaced by a carbon dioxide air atmosphere with a carbon dioxide content of at least 60% at a temperature of between 30 and 40°C and
g) the carbon dioxide air atmosphere is kept at this temperature for a period of a few hours to a few days.

2. Process according to claim 1, characterised in that the carbon dioxide air atmosphere having a carbon dioxide content of 60% at least over all is brought to a temperature of between 32 and 38°C and is kept at this temperature for one and a half to two and a half days.

## Revendications

1. Procédé pour la désinfestation de bâtiments pour le stockage et/ou le traitement de produits organiques, en particulier de moulins, dans lequel
a) on calfeutre l'espace intérieur du bâtiment vis-à-vis de l'atmosphère extérieure;
b) on porte l'air contenu dans le bâtiment à une température élevée;
c) on introduit du dioxyde de carbone dans l'espace intérieur du bâtiment;
d) on supprime le calfeutrage du bâtiment et l'on ventile l'espace intérieur du bâtiment,
caractérisé en ce que
e) l'air contenu dans le bâtiment est porté à une température comprise entre 35 et 50°C et maintenu à cette température jusqu'a ce que les murs intérieurs du bâtiment atteignent une température d'au moins 27°C;
f) l'air contenu dans le bâtiment est remplacé par une atmosphère d'air contenant du dioxyde de carbone en une proportion d'au moins 60%, d'une température comprise entre 30 et 40°C et
g) l'atmosphère d'air contenant du dioxyde de carbone est maintenue à cette température pendant une période de quelques heures à quelques jours.

2. Procédé selon la revendication 1, caractérisé en ce que l'atmosphère d'air contenant du dioxyde de carbone en une proportion globale d'au moins 60% est portée à une température comprise entre 32 et 38°C et maintenue à cette température pendant un jour et demi à deux jours et demi.
